# EUROPEAN PATENT SPECIFICATION

(11) **EP 2 077 822 B1**
(45) Date of publication and mention of the grant of the patent: **25.04.2018**
(21) Application number: 07815703.9
(22) Date of filing: 27.09.2007
(51) Int. Cl.: A61K 9/14, A61K 31/16, A61K 31/165, A61K 31/55

(54) **OXCARBAZEPINE-CONTAINING ORAL FORMULATION AND A PROCESS TO OBTAIN THE SAME**
OXCARBAZEPIN ENTHALTENDE ORALE FORMULIERUNG UND VERFAHREN ZU IHRER GEWINNUNG
FORMULATION ORALE CONTENANT OXCARBAZÉPINE ET SON PROCÉDÉ DE PRODUCTION

(30) Priority: 27.09.2006 BR PI0604387
(43) Date of publication of application: 15.07.2009
(73) Proprietor: Medley S.A. Indústria Farmacêutica, 13080-180 Campinas - SP (BR)
(72) Inventor: NEGRÃO, Alexandre Funari, Campinas - SP (BR)
(74) Representative: Vossius & Partner Patentanwälte Rechtsanwälte mbB
(86) International application number: PCT/BR2007/000261
(87) International publication number: WO 2008/037044

(56) References cited:
- EP-A1- 0 468 392
- WO-A1-03/101430
- WO-A1-2004/026314
- WO-A1-2006/046105
- WO-A1-2007/089247
- WO-A2-2007/007182
- US-A1- 2003 190 361
- COLLINS R J ET AL: "EXTENDED RELEASE FORMULATIONS OF ANTICONVULSANT MEDICATIONS: CLINICAL PHARMACOKINETICS AND THERAPEUTIC ADVANTAGES", CNS DRUGS, ADIS INTERNATIONAL, AUCKLAND, NZ, vol. 14, no. 3, 1 September 2000 (2000-09-01), pages 203-212, XP009023436, ISSN: 1172-7047, DOI: 10.2165/00023210-200014030-00003
- WALKER M C ET AL: "CLINICAL PHARMACOKINETICS OF NEW ANTIEPILEPTIC DRUGS", PHARMACOLOGY AND THERAPEUTICS, ELSEVIER, GB, vol. 67, no. 3, 1 January 1995 (1995-01-01), pages 351-384, XP001156793, ISSN: 0163-7258, DOI: 10.1016/0163-7258(95)00021-6
- MURA P. ET AL.: 'Investigations of the effects of grinding and co-grinding on physiochemical properties of glisentide' J. OF PHARM. AND BIOMED. ANALYSIS vol. 30, 2002, pages 227 - 237, XP008109648
- YAMADA T. ET AL.: 'Effects of grinding with hydroxypropyl cellulose on the dissolution and paricle size of a poorly water-soluble drug' CHEM PHARM BULL (TOKIO) vol. 47, no. 9, September 1999, pages 1311 - 1313, XP008109649

## Description

### FIELD OF THE INVENTION

The present invention is related to the Oxcarbazepine tablets formulations as well as the processes for the production of those formulations, presenting high bioavailability and low prevalence of adverse effects at the same time, using co-micronized active ingredients in a range making the intestinal absorption easier, in a modulated form. It means, the formulation reduces the adverse effects of the patients in a significant way, and at the same time does not causes damages to the crystalline structure of this active ingredient, with the preservation of the surface electrical properties of these active ingredients.

### BACKGROUND OF THE INVENTION

Oxcarbazepine is an anti-epilepsy drug, available in the form of tablets and oral suspension. Oxcarbazepine is the 10,11-dihydro-10-oxo-5H-dibenz[b,f]azepine-5-carboxamide, with molecular weight of 252.27.

The pharmacological activity of Oxcarbazepine is primary executed by it metabolite 10-monohydroxide (MHD). The exact mechanism through which Oxcarbazepine and MHD exert their anti-epilepsy effect is unknown, however, electrophysiology studies *in vitro* indicate that they cause the blocking of voltage sensitive sodium channels, resulting in the stabilization of hyperexcited neuronal membranes, inhibiting the repetitive neuronal trigger and decreasing the widespreading of sinaptic impulses. These actions are considered as being important in the prevention of the widespreading of the crisis in the intact brain. Further, the increased potassium conductance and the modulation of calcium channels activated with high voltage may can contribute in a significant way for the anticonvulsivant effects of the drug.

Oxcarbazepine and it active metabolite MHD presented anticonvulsivant properties in animal models of epilepsy. They protected the rodents against electrically induced tonic extension crisis and, in a lower grade, chemically induced chronic crisis, and they excluded or reduced the frequency of chronically recurrent focal crisis in *Rhesus* monkeys with aluminum implants, according to Schmutz and colleagues (Oxcarbazepine: preclinical anticonvulsant profile and putative mechanisms of action. Epilepsia, volume 35 Supplement 5:S47-50,1994).

After the oral administration, Oxcarbazepine is completely absorved and extensively metabolized to it pharmacologically active metabolite MHD. The shelf-life of the original drug is of approximately 2 hours, whereas the shelf-life of MHD is of approximately 9 hours, so that MHD is responsible for the largest part of the antiepileptic activity.

The use of the drug in continuous process by patients being susceptible to a setting of convulsions avoid or reduce in a significant way the occurrence of this severe setting, usually related to an epilepsy diagnosis. However, the constant use presents the possibility of undesirable side effects, such as diplopia, dysarthria, ataxia and, particularly, dizziness.

So being, the American patent US 6,296,873 from Katzhendler and Friedman claims a release form of zero order, it means, without initial release peak for anticonvulsivant drugs, including Oxcarbazepine, in the form of coated tablets of the osmotic pump type and mentions scientific works, here also incorporated as reference, proving the direct correlation between these peaks and presence of adverse effects in the central nervous system ((Epilepsia, 28:507-514 (1987); Epilepsia, 28:286-299 (1987); Epilepsia, 21:341-350 (1980); Epilepsia, 25:476-481 (1984) and Arch. Neurol., 41:830-834 (1984)). Thereafter, the increased occurrence of these side effects was also reported with the use of Oxcarbazepine particles of reduced size in comparison to the non-micronized formulation (P.M. Edelbroek and colleagues Change in Oxcarbazepine (Trileptal®) formulation is associated with more side effects and higher blood concentrations - J Neurol Neurosurg Psychiatry 2001, (71) 798-709).

The use of micronization, it means, the mechanical reduction by milling of medium size particles, is used to increase the bioavailability, it means, the absorption capacity of the drug by the gastrointestinal tract, because the contact area with the external *millieu* is increased, and, as consequence, promoting a larger absorption.

However, the micronization is a technique that must be accurate, because the determination of the exact size of the particle is an essential condition for the proper biological activity. In proper values, the micronization warrants the gain of activity in certain drugs, particularly in those being insoluble in aqueous environment, like in the intestinal *millieu.* The micronization in excess results in the breakdown of the crystalline structure of Oxcarbazepine and reduced average sizes can also accelerate the absorption in excess of the drug, with peaks of high plasmatic concentration.

Further, in micronized powders, the particles surfaces have not the same behavior of cleaved crystals, it means, according to the interfaces with lower energy. Also, micronized surfaces can present electrostatic load with clustering tendency (according to Rasenack & Muller - Micron-Size Drug Particles: Common and Novel Micronization Techniques, Pharmaceutical Development and Technology Vol 9. No 1, pages 1-13, 2004).

The micronization process can cause the disruption of the crystalline array on the particle surface creating defects.

Those defects have a tendency to present affinity for water looking for the electrostatic neutrality, explaining the increased absorption for originally quite non-soluble drugs.

The disadvantage of the processes using simple mechanical ways to obtain micronized active ingredients is that there is a reduced possibility to control important characteristics, such as the size, morphology, surface properties and electrostatic load. The particles surfaces of mechanically micronized powders, without any other additional care, are cleaved in areas usually containing less energy (Roberts,RJ and colleagues - The relationship between indentation hardness of organic solids and their molecular structure - J Mater Sci (29), pages 2289-2296,194).

As a consequence, the properties of the mechanically micronized product without additional care are those of the face of the original crystallized form. In the case of molecules being insoluble or quite insoluble in water, like Oxcarbazepine, the newly created surface continues being hydrophobic and poorly affected by the aqueous environment. Due to the aerophilicity of these hydrophobic surfaces, the dissolution speed is not increased as expected by the law of Noyes-Whitney. So being, if the drug, for instance Oxcarbazepine, is mechanically micronized to increase it bioavailability. The new surface must, by her turn, be hydrophilized. (according to Rasenack & Muller - Micron-Size Drug Particles: Common and Novel Micronization Techniques, Pharmaceutical Development and Technology Vol 9. No 1, pages 1-13, 2004) .

The co-micronization of the drug with surfactants or high solubility excipients increases the suspension bioavailability and dispersion capability in a significant way.

The bioavailability of a drug is classically defined as the fraction of the total administered reaching the systemic circulation of a living body. For poorly soluble drugs, like Oxcarbazepine, the fundamental factor related to the absorption process is the absorption rate. This factor can be dimensioned according to the Law of Noyes & Whitney, establishing that dc/dt=KaS(Cs-Ct), where dc/dt is the dissolution rate of a drug in powder, Ka is the Constant of the dissolution conditions, S is the contact area of the particle with the solvent, Cs is the drug saturation concentration and Ct is the concentration of the dissolved quantity at the time of the measurement. So being, it is estimated, according to Chaumeil in Micronization: A Method for Improving the Bioavailability of Poorly Soluble Drugs, Meth Find Exp Clin Pharmacol, vol. 20(3), pages 211-215, 1998, that the dissolution rate is directly proportional to the surface area parameter, maintaining all other conditions constant. It is licit to suppose, according to the author, that the absorption rate can be modulated, as desired, by the variation of the surface area.

The occurrence of adverse effects and serious adverse effects is commonly associated to high concentration peaks obtained in a short time period. These effects occur with larger intensity and frequency in drugs presenting an effective therapeutic concentration slightly lower than the toxic concentration. In this type of drug, also known as with narrow therapeutic window, like Oxcarbazepine, the occurrence of increased concentration, as well as of adverse effects, can be modulated by proper micronization of the raw material. The micronization in excess, even not destroying the crystalline structure of the active ingredient, can promote excessively rapid absorption in the narrow therapeutic drugs, with the occurrence of side effects with different intensities.

The adverse effects of Carbamazepine derivate drugs, like Oxcarbazepine, are originated from variations of peaks and troughs of the plasmatic concentration.

Several patent requests were submitted to improve this aspect, based on the active ingredient release control, it means, of Oxcarbazepine. So being, in the submission PCT/EP03/10475, Wolf and colleagues informs that slow release formulations can reduce these variations. In the same way, Hanshermann (PCT/EP03/05116) informs that the plasmatic concentration peaks must be avoided, obtaining a minimum Constant concentration with biologic activity.

The modified release processes, as those mentioned above (PCT/EP03/10475 and PCT/EP03/05116) are challenged with the difficulty to elaborate controlled release tablets using micronized raw-material in the lower range, it means, from 2 to 20 microns.

Therefore, the determination and use of the exact range and of the proper micronization process are fundamental conditions for each type of drug.

At the beginning of the decade of '90, the use of Oxcarbazepine does not caused serious adverse side effects, but it was necessary to take the drug together with food, to improve the absorption. The size of the particles being used was larger than 70 microns, as observed in the raw-material Oxcarbazepine without mechanical micronization treatment, being necessary to take them with food, because the increased size particles are solubilized and involved by the food bolus, as described by Flesh and colleagues (Oxcarbazepine final market image tablet formulation bioequivalence study after single administration and at steady state in healthy subjects - Int J Clin Pharmacol Ther, vol. 40(11) pages 524-32, 2002).

To improve the absorption, patent EP 98/00794 presents as main claim the micronization of Oxcarbazepine with an average particle size between 2 and 10 microns, preferentially between 4 and 10 microns, with maximum residue on 40 microns sieve of 5%, preferentially 2%. This micronization range is used by the medicine product Trileptal® from the Swiss company Novartis.

However, in this particle size range, there is a high number of adverse side effects, as reported by P.M. Edelbroek and colleagues (Change in Oxcarbazepine (Trileptal®) formulation is associated with more side effects and higher blood concentrations - J Neurol Neurosurg Psychiatry 2001, (71) 798-709). This work compared data from Trileptal® with large particles and after the micronization, as claimed in patent BR 9807368-0, observing Cmax values of MHD of 38.4 (37.5-39.6) micrograms/milliliter, but with adverse side effects like diplopia, dysarthria, ataxia, and particularly, dizziness.

This behavior of the drug Oxcarbazepine is explained by the increase of the solubilization area, promoting the extremely rapid absorption of the active ingredient, derived from the presence of defects as consequence of the mechanical process of reduction of the average size of the particles..

In this direction, Tebb & Tobias (New anticonvulsivants - New adverse effects, South Med J 99(4):375-379, 2006) reported the case of a patient who presented the side effect of hypothermia with the administration of Oxcarbazepine. According to the authors, this effect occurred due to the high level of the drug in the patient body with 22 months of age, considering that in former assessments, made on 2026 patients, this side effect was not observed.

The document WO2006046105 considers the use of Oxcarbazepine in the range of 14 to 30 microns, preferentially of 14 to 25 microns, but does not includes any innovative process protecting the active ingredient from the alterations caused by the mechanical process of particles reduction, neither against the clustering caused by this simple process, therefore without real advantages from these claims.

The decrease of the particles size by simple mechanical process is essentially a process without control. The surfaces are electrostatically loaded, and the commonly present easy clustering due to their cohesion factor. Due to the significant increase of the Van der Waals forces, the well oriented surface of the crystals disappears in the simple mechanical process, with no warranty of effective increase of hydrophilic properties.

In spite of the existence of known formulations of Oxcarbazepine, it is always desirable to improve the bioavailability with the decrease of side effects, which can be obtained with improvements associated to the range and to the co-micronization process, allowing a larger control of the particles integrity of the active ingredient, ,it means, of the Oxcarbazepine.

### OBJECTIVES AND ADVANTAGES OF THE INVENTION

Therefore, the present invention introduces, in an innovative way, a selective micronization range with efficient clinical performance, decreasing the occurrence of undesirable side effects and without structural destruction of the active ingredient, it means, of Oxcarbazepine and that can be used in coated tablets of immediate or modified release, by means of the co-micronization process together with a co-micronizing agent, as for instance, lactose of microcrystalline cellulose. The co-micronization range claimed herein presents improvements in relation to the raw-material without micronization, that must be taken with foods only, and also on lower simple micronization ranges, that, by not delaying the release, can frequently promote undesirable adverse events and being significant but more susceptible to uncontrollable processes creating amorphous areas and spontaneous re-crystallization .

### SUMMARY OF THE INVENTION

One of the objectives of the invention is to provide a pharmaceutical form of oral release or pharmaceutical compound, as well as the process to obtain them, comprising a core consisting of particles containing one or more anticonvulsivant agents co-micronized with other co-micronizing agents and pharmaceutically acceptable excipients, presenting medium size particles that may vary between approximately 15 and 40 microns, with a ratio between co-micronizing / anticonvulsivant agents that may vary between approximately 3:1 to approximately 0,5:1 and external coating with components increasing their hydrophilicity.

Other concretization of the invention comprises a process to obtain the pharmaceutical form for oral use, characterized by the fact to comprise:
(a) The co-micronization of one or more anticonvulsivant agents with one or more co-micronizing agents and pharmaceutically acceptable excipients, forming particles which average diameter can vary between approximately 15 and 40 microns, with a ratio between co-micronizing / anticonvulsivant agents that can vary between approximately 3:1 to approximately 0,5:1 ;
(b) Formation of the core composed by the particulate obtained in step (a)
(c) External coating of the core obtained, with components increasing the hydrophilicity of the pharmaceutical form.

### BRIEF DESCRIPTION OF THE DRAWINGS

Figure 1: Shows the result obtained with the co-micronization process of Oxcarbazepine used in the formulation of the tablets.
Figure 2: Shows the dissolution profile of the tablet coated with a mixture of excipients allowing the release of micronized Oxcarbazepine up to 1 hour after the administration.

### DETAILED DESCRIPTION OF THE INVENTION

Formulations were discovered in the present invention that, at the same time, are increasing the bioavailability and reducing the side effects of the continuous use of the related drug with the use of co-micronized Oxcarbazepine in coated tablets of immediate release and coated tablets of modified release.

According to the present invention, the formulation may contain different components, like pharmaceutical excipients of acceptable use that are usually used in the production of different pharmaceutical formulas, like coated tablets, for instance, for immediate or modified release.

The invention provides, in a preferred modality, the process for the production of coated tablets for immediate release, it means, with complete dissolution in less than 1 hour, and modified release, it means, for release periods of 1 to 24 hours, preferentially from 8 to 20 hours, using co-micronized Oxcarbazepine in the range of 15 to 40 microns of average particles size, preferentially between 20 and 30 microns of average particles size.

In this micronization range, with the use of other products for co-micronization and consequent increase of the bioavailability and dispersion capability, the occurrence of alterations of the crystalline structure is deniable, avoiding defects forming processes, as reported by Rasenack & Muller - Micron-Size Drug Particles: Common and Novel Micronization Techniques, Pharmaceutical Development and Technology Vol 9. No 1, pages 1-13, 2004.

The co-micronization consists of an ultrafine milling process of powders through air jet mills. Milling is executed inside a chamber of the equipment where the compressed air increases the particles speed, resulting in a collision between them, decreasing their dimensions until reaching the specified size. This is controlled in the equipment by means of the flow / pressure parameters of the compressed air. The equipments of Tecnologia Meccanica SRI, RETSH, Microservice may be used in the co-micronization because they use the technique mentioned above, among others.

The processes mentioned above differ in the addition of one or more micronizing agents together with the active ingredient to be reduced in the co-micronization. Examples of co-micronizing agents are sodium lauryl sulphate, colloidal silicon dioxide, lactose, among others. They will be adjuvant in the stabilization of the electrostatic load of the micronized powder and to increase the solubility of it.

The claimed range, it means, from 15 to 40 micron, preferentially between 20 and 30 microns, can be easily obtained by usual co-micronization processes. Therefore, combining the drug with other excipients, the clustering of the particles is avoided, resulting in their bioavailability..

The determination of this co-micronization range was obtained calculating the clinically acceptable absorption rate, following the equation of Noyes & Witman, as generically recommended by Chaumeil in Micronization: A Method for Improving the Bioavailability of Poorly Soluble Drugs, Meth Find Exp Clin Pharmacol, vol. 20(3), pages 211-215, 1998.

The co-micronization process can be obtained with the mixture of high solubility surfactant agents with the active ingredient, with a ratio of 3:1 to 0.5:1 of the co-micronizing agent; active ingredient, preferentially 1:1, it means, equal parts between the active ingredient, for instance Oxcarbazepine, and the co-micronizing agent, that can be selected from a group consisting of lactose, microcrystalline cellulose, sodium bicarbonate, potassium bicarbonate, copper carbonate, zinc carbonate, magnesium carbonate, barium carbonate, manganese carbonate, calcium silicate, magnesium silicate, copper silicate, manganese, silicate, titanium dioxide, zinc dioxide, zinc oxide, silicon oxide, aluminum oxide, magnesium oxide, zircon oxide, beryllium oxide, aluminum phosphate and their mixtures. Preferentially, the co-micronizing agent is microcrystalline cellulose with fine and medium particle size.

The co-micronizing agents act as friction elements, improving the mechanical milling conditions, and also coating the exposed surfaces of the hydrophobic drugs, protecting them against the aggregation of particles.

The co-micronizing process can be easily obtained in conventional equipments, like those from Microservice, RETSH, Tecnologia Meccanica SRI; by means of specialized companies, as well as at the raw-materials suppliers, according to the specifications established in the claims of the present submission.

On the limits of the scope of the description of the invention, the expression "coated tabled with immediate or extended release" means a solid pharmaceutical form to be orally administered, that can be manufactured by physical compression of the active ingredient, it means, of one or more carbamazepine derived anticonvulsivant agents such as Oxcarbazepine, and of the excipients, it means, the set of substances providing shape, compressibility, mechanical resistance and that modulate the release of the active ingredient. The core, it means, the set formed by the excipients and by at least one of the co-micronized anticonvulsivant agent like, for instance, a carbamazepine derived agent like Oxcarbazepine, is coated with a film providing the insulation of the core in relation to the atmospheric air, being dissolved in contact with the gastrointestinal environment.

The set of core plus coating presents a release behavior of the active ingredient in an immediate way, it means, starting from the contact with the gastrointestinal environment up to 1 hour after the administration, or even in a modified way, it means, from 1 to 24 hours after the administration of the tablet.

The set of core plus coating allows the stability of the active ingredient for a minimum period of 2 years. The stability can be defined as the absence of degradation at legally acceptable limits, it means, presenting an active ingredient content of 90 to 110% in a minimum period of 1 year..

Cores can be manufactured by dry or wet way. For the dry way, it means, through direct compression, conventional methods to compact the mixture of powders are used, by means of mechanical punch with axial force.

The excipients used for the compression are preferentially diluents, like starch of different origins like, for instance, from corn, microcrystalline cellulose like, for instance, the product Avicel®, silicon dioxide like, for instance, Aerosil®, manitol, lactose and polyethylene glycol with molecular weight of 400 to 6000, polyvinyl pyrrolidone like, for instance, Polyplasdone®, carboxymethylcellulose, hypromellose, carboxymethylamide, dicalcium phosphate, talc; agglutination agents like gelatin, alginate, cellulose ethers like, for instance, CMC and HPMC, polyethylene glycol, polyvinyl pyrrolidone, povidone, carbomer; and lubricants like magnesium stearate, calcium stearate, talc, hydrogenated vegetal oil, colloidal silicon dioxide or it mixtures. Also, different staining agents can be added, particularly to differentiate between different strengths.

The compression can be executed on rotating compression machines like, for instance, FETTE® and the shape of the cores can be diverse as well as their size, with biplanar convex, cylindrical, oblong forms.

The granules can be manufactured, for instance, with the support of silicon dioxide, microcrystalline cellulose, like Avicel®, for instance, and other like carbohydrates and starch.

The granules can be manufactured in the known way, using wet way methods. The mass, composed, for instance, of the active ingredient and microcrystalline cellulose and colloidal silicon dioxide, is mixed in a rotating mill. Thereafter, the mass is transferred to the GLATT® granulator, for instance, adding solvents selected between ethylic alcohol, isopropyl alcohol, water or their mixtures, and povidone, up to their complete dispersion. The granules are dried, placing the mass composed by Oxcarbazepine and excipients described above in a fluidized bed, and allowing the compression, that can be execute on compression machines, like FETTE® for instance. Usual compression techniques are described in Remington: The Science and Practice of Pharmacy, 19th ed. Vol. 11 (1995) (Mack Publishing Co., Pennsylvania) and Remington's Pharmaceutical Sciences, Chapter 89, pages 1633-1658 (Mach Publishing Company, 1990),

The coating looks to preserve the stability of the active ingredient, like Oxcarbazepine, due to the protection offered by the insulation of the core related to the external environment. Preferentially, the coating is permeable to water and to the humid environment of the gastrointestinal tract.

The materials composing the coating are, for instance, mixtures of polyvinyl pyrrolidone or of the copolymer of polyvinyl pyrrolidone and polyvinyl acetate with hydroxypropylmethylcellulose (HPMC) or mixtures of cellulose derivates like hydroxypropylmethylcellulose and ethylcellulose. The dispersion of these polymers is made in ethyl alcohol or water to be the application vehicle.

The solution of polymers in water or alcohol can receive the addition of pigments, talc or humectants.

The material composing the coating can be object of aspersion in the form of aqueous or alcoholic dispersion, using rotating coating equipments, like GLATT® or ACCELA COTA® type. The dispersion can receive the addition of plasticizers, like triethyl acetate, other phthalates or polyethylene glycol.

The pigments that can be included in the dispersion can be White like titanium dioxide and can be combined with ferrous oxide.

Oxcarbazepine is known, being an anticonvulsivant agent, described in the German *Auslegeschrift* 2011087, incorporated herein as a reference, being usually administered in unit doses of 300 and 600 mg of the active ingredient, it means, Oxcarbazepine.

The methods of analysis of the particles size allow the determination of medium or median size, using microscopy, sedimentation, Coulter counters and particularly LASER DIFRACTION.

The production of the particles size range producing absorption with low prevalence of adverse side effects may be executed on specific mechanical co-micronization equipments.

The co-micronization is the controlled grinding process through precision equipments like, for instance, from Temco Instruments®, JET-PHARMA®, MORRE-TEC® producing a grinded form of the raw-material, according to the desired amplitude, and with the proper co-micronizing agents, particularly the microcrystalline cellulose.

The micronization range being claimed of 15 to 40 microns, preferentially from 20 to 30 microns, allows the release of the active ingredient in coated tablets of immediate release, it means, up to 1 hour after the administration, or in coated tablets of modified release, it means, from 1 to 24 hours after the administration of the tablet, with proper clinical performance for the reduction and elimination of convulsion settings, particularly associated to epilepsy.

The objective of following examples is to illustrate the invention, however, being not allowed to use them to limit the effects of the invention.

### EXAMPLES

The co-micronization technique was used for the examples below, in which one or more micronizing agents are added to the active ingredient to be reduced in the co-micronization, in equipments such as those from Tecnologia Meccanica SRI, RETSH, Microservice. In the micronization process through compressed air Jet Mill the product being micronized is put in a feed thread of a micronizator. The feed thread carry the raw material to an ejector, that feeds the micronization chamber. There, the particles collide to each other due the compressed air pression. The centrifuge force causes greater particles rotation at the chamber extremity, colliding themselves. The particles that reached the requested size are led to centre of the chamber, where inside thereof exists a micronization material extractor pipe.
The compressed air passes through the chamber and emerges by this pipe until the storage silo of micronized product. The air leaves the system through a filter under the silo. There, exists a container that receives the micronized product.

The process is a continuous one and to reach the requested specification, the feed flow of raw material and the compressed air pressure are regulated. The compressed air is filtered, free of water and oil, and in a temperature between 20 and 24 degrees Celsius. During the micronization, due to the air expansion into the chamber, the temperature decreases. The already filtered compressed air leaves the micronization place by a pipe connected to an exhaust. After this, also exists a finer mesh filter, working as an anti-pollutant system. The micronization place works with negative pressure.

The process has been analyzed with the results of two readings per sample and 5 replications in the distribution measured by laser in equipment from Mastersize S Malvern model S- MAM 5005. The use of the dispersion agent Tween 20 (0,1 mL of Tween 20 for 1 Liter of distilled water). The analyzed range was of 0.005 microns to 900 microns. The results showed that 90% of the sample is comprised in the range of 20 to 30 microns.

### Example 1

| Tablet core: | (mg) | (mg) |
|---|---|---|
| Co-micronized Oxcarbazepine | 300 | 600 |
| Microcrystalline cellulose | 80.8 | 161.6 |
| Colloidal silicon dioxide | 4.8 | 9.6 |
| Magnesium stearate | 2.0 | 4.0 |
| Povidone | 6.4 | 12.8 |
| Magnesium silicate | 6.0 | 12.0 |
| | | |
| Coating: | (mg) | (mg) |
| | | |
| Opadry® | 8.0 | 24.0 |
| Yellow iron oxide | 1.0 | 2.0 |
| | | |
| Total | 409.0 | 826.0 |

Mix the co-micronized Oxcarbazepine, which co-micronizing agent is microcrystalline cellulose, both in the range of 20 to 30 microns, with half of Colloidal silicon dioxide, Povidone and half of Microcrystalline cellulose in a high speed Glatt® mixer. Add alcohol or water to the mixture as the granulation fluid, and execute the granulation up to the proper consistency. As an alternative, the Povidone can be previously dissolved in the granulation fluid. The granules are processed using a proper device, Quadro-Comill®, and dried in the Aeromatic® fluidized bed or in a Bernauer® heating stove with air circulation. Add the remaining Microcrystalline cellulose and the part of Colloidal silicon dioxide and mix. Calibrate the dry granulate. Finally, add the Magnesium stearate and the Magnesium silicate and mix. Alternatively, the lubricant can be added to the calibrated granulate. Press the final mixture of Oxcarbazepine in a rotating press Fette®.

Coat the tablets with a hydro-alcoholic preparation composed of Opadry® and Yellow iron oxide in a rotating coating pan Accela -cota®. Alternatively, it is possible to use, for instance, an equipment of Glatt® fluidized bed or air suspension for the coating process.

### Example 2

### Formulations

| Tablet core: | (mg) | (mg) |
|---|---|---|
| Co-micronized Oxcarbazepine | 300 | 600 |
| Microcrystalline cellulose | 77.9 | 155.8 |
| Colloidal silicon dioxide | 5.2 | 10.4 |
| Magnesium stearate | 2.5 | 5.0 |
| Povidone | 6.4 | 12.8 |
| Magnesium silicate | 8.0 | 16.0 |
| | | |

| Coating: | (mg) | (mg) |
|---|---|---|
| Opadry® | 8.0 | 24.0 |
| Yellow iron oxide | 1.0 | 2.0 |
| | | |
| Total | 409.0 | 826.0 |

Mix the Oxcarbazepine, Colloidal silicon dioxide, Povidone and half of the Microcrystalline cellulose obtained from the co-micronization process, in a high speed Diosna® mixer. Add alcohol or water to the mixture as granulation fluid, and execute the granulation up to the proper consistency. Alternatively, the Povidone can be previously dissolved in the granulation fluid. The granules are processed using a Quadro-Comill® device and are dried in a fluidized bed from Glatt or in a heating stove with air circulation from Bernauer®. Add the remaining Microcrystalline cellulose and mix. Calibrate the dry granulate. Finally, add the Magnesium stearate and the Magnesium silicate and mix. Alternatively, the lubricant can be added to the calibrated granulate. Press the final mixture to form Oxcarbazepine tablets in a rotating press from Fette®.

Coat the tablets with a hydro-alcoholic preparation composed of Opadry® e Yellow iron oxide in a rotating coating pan from Accela -cota®. Alternatively, it is possible to use, for instance, a Glatt® fluidized bed equipment or air suspension for the coating process.

### Example 3

### Formulations

| Tablet core: | (mg) | (mg) |
|---|---|---|
| Co-micronized Oxcarbazepine | 300.0 | 600.0 |
| Carbomer | 30.0 | 60.0 |
| Microcrystalline cellulose | 80.8 | 161.6 |
| Colloidal silicon dioxide | 4.8 | 9.6 |
| Magnesium stearate | 2 0 | 4.0 |
| Povidone | 6.4 | 12.8 |
| Magnesium silicate | 6.0 | 12.0 |
| | | |

| Coating: | (mg) | (mg) |
|---|---|---|
| Opadry® | 8.0 | 24.0 |
| Yellow iron oxide | 1.0 | 2.0 |
| | | |
| Total | 439.0 | 886.0 |

Mix the Oxcarbazepine, part of the Colloidal silicon dioxide, Povidone, Carbomer and part of the Microcrystalline cellulose from the co-micronization process, in a high speed Diosna® mixer. Add alcohol or water to the mixture as the granulation fluid and execute the granulation up to the proper consistency. Alternatively, the Povidone can be previously dissolved in the granulation fluid. Granulate the wet granules using a proper Quadro-Comill® device and dry in a Glatt® fluidized bed or in a heating stove with air circulation from Bernauer®. Add the remaining Microcrystalline cellulose and part of the Colloidal silicon dioxide and mix. Calibrate the dry granulate in the Quadro-Comill®. Finally, add the Magnesium stearate and the Magnesium silicate and mix. Alternatively, the lubricant can be added to the calibrated granulate. Press the final mixture to form the Oxcarbazepine tablets in a rotating Fette® press.

Coat the tablets with a hydro-alcoholic preparation consisting of Opadry® and Yellow iron oxide in a rotating coating pan from Accela -cota®. Alternatively, it is possible to use, for instance, a Glatt® fluidized bed equipment or air suspension for the coating process.

### Example 4

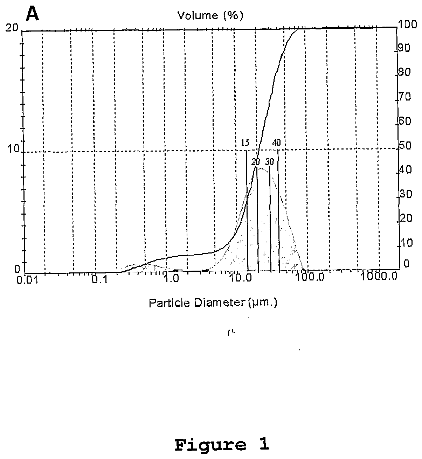

Figure 1 and **TABLE 1** below show the results obtained with the co-micronization process of Oxcarbazepine used in the formulation of the tablets, according to the invention, for an average range of particles of 25 microns. These values from a laser reading were obtained with equipment from Mastersize S Malvern model S- MAM 5005.

**TABLE 1**

| Conc.= 0.0156 %Vol) | | | Density= 1.120g/cm°3 | | S.SA= 1.1778 m°2/g | | |
|---|---|---|---|---|---|---|---|
| Distribution : Volume | | | D[4.3] = 24.46 um | | D[3,2] = 4.55 um | | |
| D(v.0.1)= 627 um | | | D(v.0.5)= 21.18um | | D(v.0.9)= 47.80 um | | |
| Span = 1.961E+00 | | | Uniformity= 6004E-01 | | | | |
| Size (um) | Volume Under% | Size (um) | Volume Under% | Size (um) | Volume Under% | Size (um) | Volume Under% |
| 0.05 | 0.00 | 0.67 | 4.97 | 9.00 | 14.84 | 120.67 | 100.00 |
| 0.06 | 0.00 | 0.78 | 5.54 | 10.48 | 18.54 | 140.58 | 100.00 |
| 0.07 | 0.00 | 0.91 | 6.01 | 12.21 | 23.45 | 163.77 | 100.00 |
| 0.08 | 0.00 | 1.06 | 6.40 | 14.22 | 29.61 | 190.80 | 100.00 |
| 0.09 | 0.00 | 1.24 | 6.71 | 16.57 | 36.86 | 222.28 | 100.00 |
| 0.11 | 0.01 | 1.44 | 6.98 | 19.31 | 44.89 | 258.95 | 100.00 |
| 0.13 | 0.02 | 1.58 | 7.23 | 22.49 | 53.33 | 301.68 | 100.00 |
| 0.15 | 0.06 | 1.35 | 7.35 | 26.20 | 61.79 | 351.46 | 100.00 |
| 0.17 | 0.13 | 228 | 7.47 | 30.53 | 69.97 | 409.45 | 100.00 |
| 0.20 | 0.28 | 2.65 | 7.61 | 35.56 | 77.68 | 477.01 | 100.00 |
| 0.23 | 0.54 | 3.09 | 7.75 | 41.43 | 84.55 | 555.71 | 100.00 |
| 0.27 | 0.96 | 3.60 | 7.94 | 48.27 | 90.33 | 647.41 | 100.00 |
| 0.31 | 1.53 | 4.19 | 8.22 | 56.23 | 94.83 | 75423 | 100.00 |
| 0.36 | 2.20 | 4.88 | 8.65 | 65.51 | 97.86 | 878.67 | 100.00 |
| 0.42 | 2.90 | 5.69 | 9.35 | 76.32 | 99.62 | | |
| 0.49 | 3.61 | 6.63 | 10.47 | 88.91 | 100.00 | | |
| 0.58 | 4.32 | 7.72 | 12.23 | 103.68 | 100.00 | | |

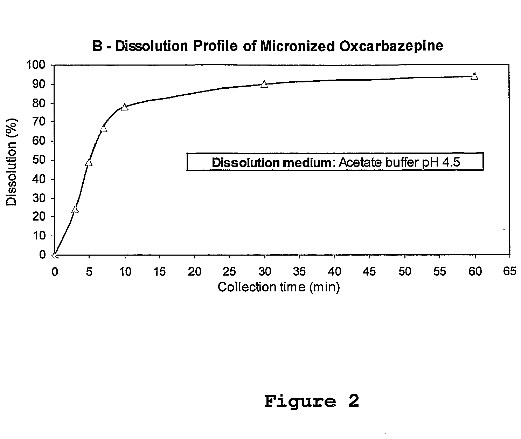

Figure 2 shows the dissolution profile of the tablet coated with a mixture of excipients allowing the release of micronized Oxcarbazepine up to 1 hour after the administration in a dissolution test *in vitro,* conducted in an Erweka equipment with 50 rotations per minute in acetate buffer pH 4.5 with addition of 0.75% of sodium lauryl sulphate.

## Claims

1. Pharmaceutical form for oral administration comprising a core consisting of particles containing at least one anticonvulsivant agent co-micronized with other co-micronizing agents and pharmaceutically acceptable excipients, presenting particles with medium size, varying between 15 and 40 microns, with a ratio between co-micronizing agents / anticonvulsivant agents that can vary between 3:1 to 0.5:1 and externally coated with coating agents which increase hydrophilicity, wherein the at least one anticonvulsivant agent is selected from carbamazepine derivates.

2. Pharmaceutical form for oral administration according to claim 1 wherein the anticonvulsivant agent is oxcarbazepine.

3. Pharmaceutical form for oral administration according to claim 1 wherein the average diameter of the particles is, preferentially, of 20 to 30 microns.

4. Pharmaceutical form for oral administration according to claim 1 wherein the pharmaceutical form is presented in the form of tablets and is for immediate release or modified release.

5. Pharmaceutical form for oral administration according to claim 1 wherein the co-micronizing agents or the excipients are selected from a group consisting of dilution agents, surfactants, lubricants, detergents and staining agents.

6. Pharmaceutical form for oral administration according to claim 5 wherein the dilution agents are selected from carbohydrates, corn starch, microcrystalline cellulose, silicon dioxide, manitol, lactose, polyethylene glycol with molecular weight of 400 to 6000, carboxymethylcellulose, hypromellose, carboxymethylamide, dicalcium phosphate, talc and their mixtures.

7. Pharmaceutical form for oral administration according to claim 5 wherein the surfactants or lubricants are the compounds of the family of the stearates, preferentially magnesium stearate.

8. Pharmaceutical form for oral administration according to claim 1 wherein the excipients comprise agglutination agents selected from gelatin, alginate, cellulose ethers like CMC and HPMC, polyethylene glycol, polyvinyl pyrrolidone, povidone, carbomer and their mixtures.

9. Pharmaceutical form for oral administration according to claim 5 wherein the detergents are selected from sodium lauryl sulphate, dodecyl sodium sulphate, or their mixtures.

10. Pharmaceutical form for oral administration according to claim 1 wherein the coating agents are selected from coatings of the group consisting of mixtures of polyvinyl pyrrolidone or of the co-polymer of polyvinyl pyrrolidone and polyvinyl acetate with hydroxypropylmethylcellulose (HPMC), mixtures of cellulose derivates like hydroxypropylmethylcellulose and ethylcellulose and their mixtures.

11. Pharmaceutical form for oral administration according to claim 5 wherein the pigments are titanium dioxide or ferrous oxide.

12. Pharmaceutical composition for oral administration comprising a core consisting of particles containing at least one anticonvulsivant agent co-micronized with other co-micronizing agents and pharmaceutically acceptable excipients, presenting particles with medium size, varying between 15 and 40 microns, with a ratio between co-micronizing agents / anticonvulsivant agents that can vary between approximately 3:1 to approximately 0.5:1 and externally coated with components increasing the hydrophilicity of the core, wherein the at least one anticonvulsivant agent is a carbamazepine derivate.

13. Pharmaceutical composition for oral administration according to claim 12 wherein the carbamazepine derivate is oxcarbazepine.

14. Pharmaceutical composition for oral administration according to claim 12 comprising the use of a mixture of excipients allowing the release of co-micronized Oxcarbazepine up to 1 hour after the administration.

15. Pharmaceutical composition for oral administration according to claim 12 comprising the use of a mixture of excipients allowing the release of co-micronized Oxcarbazepine from 1 to 24 hours after the administration.

16. Process to obtain the pharmaceutical form for oral use comprising:
(a) The co-micronization of one or more anticonvulsivant agents with one or more co-micronizing agents and pharmaceutically acceptable excipients, forming particles which average diameter can vary between approximately 15 and 40 microns, with a ratio between co-micronizing agents / anticonvulsivant agents that can vary between approximately 3:1 and approximately 0.5:1, wherein the anticonvulsivant agents are carbamazepine derivates;
(b) Production of the core formed by the particulate obtained in stage (a);
(c) External coating of the core obtained, with components increasing the hydrophilicity of the pharmaceutical form.

17. Process to obtain the pharmaceutical form for oral use according to claim 16 wherein the anticonvulsivant agent is oxcarbazepine.

18. Process to obtain the pharmaceutical form for oral use according to claim 16 wherein the cores can be manufactured by the dry or wet way.

## Patentansprüche

1. Pharmazeutische Form zur oralen Verabreichung, umfassend einen Kern, der aus Partikeln besteht, die mindestens ein antikonvulsives Agens, das mit anderen co-mikronisierenden Agenzien co-mikronisiert ist, und pharmazeutisch verträgliche Exzipienten enthalten, die Partikel mittlerer Größe darstellen, die zwischen 15 und 40 Mikron variieren, wobei das Verhältnis zwischen co-mikronisierenden Agenzien / antikonvulsiven Agenzien von 3:1 bis 0,5:1 variieren kann, und die außen mit Beschichtungsagenzien beschichtet sind, die die Hydrophilizität erhöhen, wobei das mindestens eine antikonvulsive Agens ausgewählt ist aus Carbamazepin-Derivaten.

2. Pharmazeutische Form zur oralen Verabreichung nach Anspruch 1, wobei das antikonvulsive Agens Oxcarbazepin ist.

3. Pharmazeutische Form zur oralen Verabreichung nach Anspruch 1, wobei der mittlere Durchmesser der Partikel vorzugsweise 20 bis 30 Mikron ist.

4. Pharmazeutische Form zur oralen Verabreichung nach Anspruch 1, wobei die pharmazeutische Form in Form von Tabletten vorliegt und für die sofortige Freisetzung oder modifizierte Freisetzung ist.

5. Pharmazeutische Form zur oralen Verabreichung nach Anspruch 1, wobei die co-mikronisierenden Agenzien oder die Exzipienten ausgewählt sind aus der Gruppe bestehend aus Verdünnungsmitteln, oberflächenaktiven Stoffen, Gleitmitteln, Detergenzien und Färbemitteln.

6. Pharmazeutische Form zur oralen Verabreichung nach Anspruch 5, wobei die Verdünnungsmittel ausgewählt sind aus Kohlehydraten, Maisstärke, mikrokristalline Cellulose, Siliziumdioxid, Mannitol, Laktose, Polyethylenglykol mit einem Molekulargewicht von 400 bis 6000, Carboxymethylcellulose, Hypromellose, Carboxymethylamid, Dicalciumphosphat, Talkum und deren Gemische.

7. Pharmazeutische Form zur oralen Verabreichung nach Anspruch 5, wobei die oberflächenaktiven Stoffe oder Verdünnungsmittel Verbindungen der Familie der Stearate, vorzugsweise Magnesiumstearat, sind.

8. Pharmazeutische Form zur oralen Verabreichung nach Anspruch 1, wobei die Exzipienten Agglutinationsagenzien umfassen, die ausgewählt sind aus Gelatine, Alginat, Celluloseether wie CMC und HPMC, Polyethylenglykol, Polyvinylpyrrolidon, Povidon, Carbomer und deren Gemische.

9. Pharmazeutische Form zur oralen Verabreichung nach Anspruch 5, wobei die Detergenzien ausgewählt sind aus Natriumlaurylsulfat, Natriumdodecylsulfat oder deren Gemische.

10. Pharmazeutische Form zur oralen Verabreichung nach Anspruch 1, wobei die Beschichtungsagenzien ausgewählt sind aus Beschichtungen der Gruppe bestehend aus Gemischen aus Polyvinylpyrrolidon oder des Co-Polymers von Polyvinylpyrrolidon und Polyvinylacetat mit Hydroxypropylmethylcellulose (HPMC), Gemischen aus Cellulose-Derivaten wie Hydroxypropylmethylcellulose und Ethylcellulose und deren Gemische.

11. Pharmazeutische Form zur oralen Verabreichung nach Anspruch 5, wobei die Pigmente Titandioxid oder Eisen (II)-oxid sind.

12. Arzneimittel zur oralen Verabreichung, umfassend einen Kern, der aus Partikeln besteht, die mindestens ein antikonvulsives Agens, das mit anderen co-mikronisierenden Agenzien co-mikronisiert ist, und pharmazeutisch verträgliche Exzipienten enthalten, die Partikel mittlerer Größe darstellen, die zwischen 15 und 40 Mikron variieren, wobei das Verhältnis zwischen co-mikronisierenden Agenzien / antikonvulsiven Agenzien von etwa 3:1 bis etwa 0,5:1 variieren kann, und die außen mit Bestandteilen beschichtet sind, die die Hydrophilizität des Kerns erhöhen, wobei das mindestens eine antikonvulsive Agens ein Carbamazepin-Derivat ist.

13. Arzneimittel zur oralen Verabreichung nach Anspruch 12, wobei das Carbamazepin-Derivat Oxcarbazepin ist.

14. Arzneimittel zur oralen Verabreichung nach Anspruch 12, umfassend die Verwendung eines Gemisches aus Exzipienten, die die Freisetzung von comikronisiertem Oxcarbazepin bis zu 1 Stunde nach der Verabreichung ermöglichen.

15. Arzneimittel zur oralen Verabreichung nach Anspruch 12, umfassend die Verwendung eines Gemisches aus Exzipienten, die die Freisetzung von comikronisiertem Oxcarbazepin von 1 bis 24 Stunden nach der Verabreichung ermöglichen.

16. Verfahren, um die pharmazeutische Form zur oralen Verwendung zu erhalten, umfassend:
(a) Co-Mikronisierung von einem oder mehreren antikonvulsiven Agenzien mit einem oder mehreren co-mikronisierenden Agenzien und pharmazeutisch verträglichen Exzipienten, Bilden von Partikeln, deren durchschnittlicher Durchmesser zwischen etwa 15 und 40 Mikron variieren kann, mit einem Verhältnis zwischen co-mikronisierenden Agenzien / antikonvulsiven Agenzien, das zwischen etwa 3:1 und etwa 0,5:1 variieren kann, wobei die antikonvulsiven Agenzien Carbamazepin-Derivate sind;
(b) Herstellung eines Kerns, der durch die in Phase (1) erhaltenen Partikel gebildet wird;
(c) Äußeres Beschichten des erhaltenen Kerns, mit Bestandteilen, die die Hydrophilizität der pharmazeutischen Form erhöhen.

17. Verfahren, um die pharmazeutische Form zur oralen Verwendung nach Anspruch 16 zu erhalten, wobei das antikonvulsive Agens Oxcarbazepin ist.

18. Verfahren, um die pharmazeutische Form zur oralen Verwendung nach Anspruch 16 zu erhalten, wobei die Kerne trocken oder nass hergestellt werden können.

## Revendications

1. Formulation pharmaceutique pour administration orale comprenant un coeur constitué de particules contenant au moins un agent anticonvulsivant co-micronisé avec d'autres agents co-micronisants et des excipients pharmaceutiquement acceptables, présentant des particules ayant une taille moyenne, variant entre 15 et 40 microns, avec un rapport entre agents co-micronisants/agents anticonvulsivants qui peut varier entre 3/1 et 0,5/1 et enrobées de manière externe avec des agents d'enrobage qui augmentent l'hydrophilicité, dans laquelle l'au moins un agent anticonvulsivant est choisi parmi les dérivés de carbamazépine.

2. Formulation pharmaceutique pour administration orale selon la revendication 1 dans laquelle l'agent anticonvulsivant est de l'oxcarbazépine.

3. Formulation pharmaceutique pour administration orale selon la revendication 1 dans laquelle le diamètre moyen des particules est, de préférence, de 20 à 30 microns.

4. Formulation pharmaceutique pour administration orale selon la revendication 1 dans laquelle la formulation pharmaceutique se présente sous la forme de comprimés et est destinée à une libération immédiate ou à une libération modifiée.

5. Formulation pharmaceutique pour administration orale selon la revendication 1 dans laquelle les agents co-micronisants ou les excipients sont choisis dans un groupe constitué d'agents de dilution, de tensioactifs, de lubrifiants, de détergents et d'agents de coloration.

6. Formulation pharmaceutique pour administration orale selon la revendication 5 dans laquelle les agents de dilution sont choisis parmi les glucides, l'amidon de maïs, la cellulose microcristalline, le dioxyde de silicium, le mannitol, le lactose, le polyéthylène glycol ayant un poids moléculaire de 400 à 6 000, la carboxyméthylcellulose, l'hypromellose, le carboxyméthylamide, le phosphate de dicalcium, le talc et leurs mélanges.

7. Formulation pharmaceutique pour administration orale selon la revendication 5 dans laquelle les tensioactifs ou les lubrifiants sont les composés de la famille des stéarates, de préférence du stéarate de magnésium.

8. Formulation pharmaceutique pour administration orale selon la revendication 1 dans laquelle les excipients comprennent des agents d'agglutination choisis parmi la gélatine, l'alginate, les éthers de cellulose tels que la CMC et l'HPMC, le polyéthylène glycol, la polyvinyl pyrrolidone, la povidone, un carbomère et leurs mélanges.

9. Formulation pharmaceutique pour administration orale selon la revendication 5 dans laquelle les détergents sont choisis parmi le laurylsulfate de sodium, le dodécylsulfate de sodium, ou leurs mélanges.

10. Formulation pharmaceutique pour administration orale selon la revendication 1 dans laquelle les agents d'enrobage sont choisis parmi les enrobages du groupe constitué des mélanges de polyvinyl pyrrolidone ou du co-polymère de polyvinyl pyrrolidone et d'acétate de polyvinyle avec de l'hydroxypropylméthylcellulose (HPMC), des mélanges de dérivés de cellulose tels que l'hydroxypropylméthylcellulose et l'éthylcellulose et leurs mélanges.

11. Formulation pharmaceutique pour administration orale selon la revendication 5 dans laquelle les pigments sont le dioxyde de titane ou l'oxyde ferreux.

12. Composition pharmaceutique pour administration orale comprenant un coeur constitué de particules contenant au moins un agent anticonvulsivant co-micronisé avec d'autres agents co-micronisants et des excipients pharmaceutiquement acceptables, présentant des particules ayant une taille moyenne variant entre 15 et 40 microns, avec un rapport entre agents co-micronisants/agents anticonvulsivants qui peut varier entre approximativement 3/1 et approximativement 0,5/1 et enrobées de manière externe avec des composants augmentant l'hydrophilicité du coeur, dans laquelle l'au moins un agent anticonvulsivant est un dérivé de carbamazépine.

13. Composition pharmaceutique pour administration orale selon la revendication 12 dans laquelle le dérivé de carbamazépine est de l'oxcarbazépine.

14. Composition pharmaceutique pour administration orale selon la revendication 12 comprenant l'utilisation d'un mélange d'excipients permettant la libération d'oxcarbazépine co-micronisée jusqu'à 1 heure après l'administration.

15. Composition pharmaceutique pour administration orale selon la revendication 12 comprenant l'utilisation d'un mélange d'excipients permettant la libération d'oxcarbazépine co-micronisée de 1 à 24 heures après l'administration.

16. Procédé d'obtention de la formulation pharmaceutique pour utilisation orale comprenant :
(a) la co-micronisation d'un ou plusieurs agents anticonvulsivants avec un ou plusieurs agents co-micronisants et excipients pharmaceutiquement acceptables, la formation de particules dont le diamètre moyen peut varier entre approximativement 15 et 40 microns, avec un rapport entre agents co-micronisants/agents anticonvulsivants qui peut varier entre approximativement 3/1 et approximativement 0,5/1, dans lequel les agents anticonvulsivants sont des dérivés de carbamazépine ;
(b) la production du coeur formé par la substance particulaire obtenue à l'étape (a);
(c) l'enrobage externe du coeur obtenu, avec des composants augmentant l'hydrophilicité de la formulation pharmaceutique.

17. Procédé d'obtention de la formulation pharmaceutique pour utilisation orale selon la revendication 16 dans lequel l'agent anticonvulsivant est de l'oxcarbazépine.

18. Procédé d'obtention de la formulation pharmaceutique pour utilisation orale selon la revendication 16 dans lequel les coeurs peuvent être fabriqués par voie sèche ou humide.
